# EUROPÄISCHE PATENTANMELDUNG

(11) **EP 4 122 512 A1**
(43) Veröffentlichungstag der Anmeldung: **25.01.2023**
(21) Anmeldenummer: 21187112.4
(22) Anmeldetag: 22.07.2021
(51) Int. Cl.: A61M 5/20, A61M 5/32

(54) **AUTOINJEKTOR MIT TRENNBAREM ELEKTRONIKMODUL**

(71) Anmelder: Ypsomed AG, 3401 Burgdorf (CH)
(72) Erfinder: URBANEK, Leos, 3012 Bern (CH); KALBERMATTER, Gabriel, 3400 Burgdorf (CH); TSCHIRREN, Markus, 3400 Burgdorf (CH); ZUMSTEIN, Dominik, 3014 Bern (CH)
(74) Vertreter: Meier Obertüfer, Jürg

(57) **Zusammenfassung**

Die Erfindung betrifft ein Injektionsgerät zur subkutanen Verabreichung eines maximalen Inhalts eines Produktbehälters durch eine Kanüle an einem distalen Ende des Produktbehälters, umfassend ein Gerätegehäuse (2) mit einer Öffnung an einem proximalen Ende, ein Modulgehäuse (17) zur Abdeckung der Öffnung, welches von dem Gerätegehäuse durch einen Trennvorgang trennbar ist, einen in dem Gerätegehäuse angeordneter Antrieb (9, 13) zur Bewegung eines Vortriebsglieds (7) und zur einmaligen automatischen Ausschüttung des Inhalts des Produktbehälters. In dem Modulgehäuse ist ein Elektronikmodul (16) gehalten mit einem Sensor (16a) zur Detektion der Ausschüttung, und ein mechanischer Sicherungsmechanismus (19) ist vorgesehen zur Freigabe des Trennvorgangs mit oder nach der Auslösung der automatischen Ausschüttung.

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf einen Autoinjektor mit einem Energiespeicher zur Ausschüttung einer vorgegebenen Dosis aus einem einmalig genutzten Produktbehälter.

### HINTERGRUND DER ERFINDUNG

Injektionsgeräte oder Injektionsvorrichtungen zum vereinfachten Verabreichen einer Substanz umfassen unter anderem so genannte Autoinjektoren, welche einen Energiespeicher aufweisen, mit welchem die Ausschüttung automatisch, das heisst ohne extern von einem Benutzer zuzuführende oder aufzuwendende Kraft, durchgeführt werden kann. Der Energiespeicher speichert die für eine automatische Substanzabgabe erforderliche Energie vorteilhaft in mechanischer Form. Ein solcher Energiespeicher kann eine Feder sein, welche in einem gespannten Zustand in das Injektionsgerät eingebaut wird und durch Entspannen Energie abgibt. Die Energieabgabe erfolgt an eine Kolbenstange oder ein Druckelement, welches einen Kolben in einen Produktbehälter einschiebt. Der Energiespeicher kann auch vorgesehen sein um den Vorgang des Einstechens einer Injektionsnadel zu automatisieren. Alternativ kann der Einstechvorgang manuell erfolgen, also ausschliesslich durch einen Benutzer, ohne hierfür in dem Injektionsgerät gespeicherte Energie zu verwenden.

Das Injektionsgerät kann einen Produktbehälterhalter zur Aufnahme eines Produktbehälters umfassen, wobei in dem Produktbehälterhalter der Produktbehälter radial, axial und vorzugsweise auch drehfest gehalten werden kann. Der Produktbehälterhalter kann mit dem Gehäuse der Injektionsvorrichtung axial- und drehfest verbunden sein, oder bei einem Einstech- und/oder Nadelrückzugsvorgang relativ zum Gehäuse bewegbar sein. Der Produktbehälter kann eine Karpule zur wiederholt lösbaren Verbindung mit Einweg-Injektionsnadeln oder eine Einweg-Fertigspritze mit einer damit unlösbar verbundenen Injektionsnadel sein. Der Produktbehälter weist einen hohlzylindrischen Produktbehälterabschnitt auf, der einen Kolben oder Stopfen verschiebbar lagert. Der Kolben kann mit dem Innenumfang des Produktbehälterabschnitts einen Dichtspalt bilden und mittels Kolbenstange in eine distale Richtung verschoben werden, um über die Injektionsnadel Produkt aus dem Produktbehälter abzugeben.

Das Injektionsgerät kann eine Nadelschutzhülse aufweisen, die nach erfolgter Injektion distal über das distale Ende der Injektionsnadel steht oder relativ zu dem Gehäuse unter Entspannung einer Nadelschutzhülsenfeder in diese Position verschoben wird, um den versehentlichen Zugriff auf die Injektionsnadel zu verhindern und dadurch ein Verletzungsrisiko zu verringern. Bei einem Autoinjektor kann die Nadelschutzhülse auch als Auslöseelement zum Auslösen der Produktausschüttung dienen, wobei die Nadelschutzhülse hierzu relativ zu dem Gehäuse in die proximale Richtung verschoben wird. Alternativ kann die Auslösung des Autoinjektors durch Betätigen eines Auslöseknopfs des Autoinjektors erreicht werden, wobei die Nadelschutzhülse vor dem Gebrauch des Autoinjektors zumindest als Sichtschutz dient.

Die Patentanmeldung PCTEP2021057168 beschreibt einen Autoinjektor umfassend ein Gehäuse mit einer Längsachse, einen Produktbehälter, eine zur einmaligen Ausschüttung eines maximalen Inhalts des Produktbehälters vorgespannte Torsionsfeder, ein Antriebselement, und ein Vortriebselement. Zur Ausschüttung einer maximalen oder zumindest einer vorbestimmten Flüssigkeitsmenge aus dem Produktbehälters versetzt die Torsionsfeder das Antriebselement in Rotation um die Längsachse, und das rotierende Antriebselement bewirkt eine Vortriebsbewegung des Vortriebselements zur Verschiebung eines Kolbens im Produktbehälter. Der Autoinjektor umfasst weiter einen permanent eingebauten Rotationssensor zur Detektion eines Starts der Ausschüttung. Der Autoinjektor umfasst eine Kommunikationseinheit zur Kommunikation mit einem mobilen oder stationären Drittgerät und/oder eine Anzeigeeinheit zur Anzeige eines Zustandes des Autoinjektors. Die Kommunikationseinheit und/oder die Anzeigeeinheit, und eine Energiequelle zu deren Speisung sind auf einer Leiterplatine angeordnet, welche zusammen mit einem bevorzugt durch vorgegebene Sollbruchlinien im Gehäuse einfach abtrennbaren Gehäuseteil vom Rest des Autoinjektors entfernt werden kann. Bevorzugt sind auch alle entsorgungskritischen elektronischen Komponenten des Rotationssensors auf der Leiterplatine angeordnet, so dass keine elektronischen Bauteile nach Entfernen der Leiterplatine im Autoinjektor zurückbleiben.

Die Patentanmeldung WO 2017/129314 beschreibt einen Autoinjektor mit einem Detektor zur Detektion eines Starts einer Ausschüttung basierend auf einer Bewegung eines Auslöseglieds welches durch eine Nadelschutzhülse beim Aufsetzen des Autoinjektors auf die Einstichstelle nach proximal gedrückt wird. Durch die erfolgte Detektion wird eine Kommunikationseinheit des Autoinjektors aktiviert. Eine proximale Bewegung eines Endklickelements am Ende der Ausschüttung wird durch einen zweiten Detektor detektiert. Die Kommunikationseinheit ist unlösbar mit dem Autoinjektor verbunden oder Teil eines durch den Anwender selbst mit dem Autoinjektor verbindbaren Zusatzmoduls.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder coformulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Verabreichungsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Verabreichungsvorrichtung hin gerichtete Seite oder Richtung.

Unter den Begriffen "Injektionssystem" oder "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektionssystem oder bei einem Injektor im Unterschied zu einem Infusionssystem die Injektionsnadel nicht über einen längeren Zeitraum von mehreren Stunden im Gewebe.

### DARSTELLUNG DER ERFINDUNG

Es ist Aufgabe der Erfindung ein Injektionsgerät zum automatischen Ausschütten einer Flüssigkeit aus einem Produktbehälter anzugeben mit einem zur gesonderten Entsorgung sicher trennbaren Elektronikmodul. Die Aufgabe wird gelöst durch ein Injektionsgerät mit den Merkmalen der unabhängigen Ansprüche. Bevorzugte Ausführungsformen der Erfindung sind Gegenstand der abhängigen Ansprüche.

Erfindungsgemäss umfasst das Injektionsgerät zur subkutanen Verabreichung eines maximalen oder gesamten Inhalts eines Produktbehälters, insbesondere einer Dosis eines flüssigen Medikaments, durch eine Kanüle oder Injektionsnadel an einem distalen Ende des Produktbehälters folgende Komponenten:
- Ein Gerätegehäuse mit einem Griffbereich zum Ergreifen und Halten des Injektionsgeräts durch einen Anwender während der Verabreichung, definierend eine Längsrichtung, und mit einer Öffnung zu Montagezwecken an einem proximalen Ende.
- Ein Modulgehäuse oder eine Modulkappe zur Abdeckung oder zum Abschluss der Öffnung des Gerätegehäuses, welches von dem Gerätegehäuse durch einen Trennvorgang eines Anwenders trennbar ist.
- Ein in dem Gerätegehäuse angeordneter Antrieb mit einem Energiespeicher in Form einer vorgespannten Ausschüttfeder zur Bewegung eines Vortriebsglieds in Längsrichtung und zur einmaligen automatischen und bevorzugt nicht-elektrischen Ausschüttung des Inhalts des Produktbehälters.
- Ein in dem Modulgehäuse gehaltenes Elektronikmodul mit einem Sensor zur Detektion eines Vorganges der Ausschüttung, und mit einer Kommunikationseinheit zur drahtlosen Übermittlung eines Berichts über den detektierten Vorgang der Ausschüttung. Das Elektronikmodul umfasst zumindest alle entsorgungstechnisch relevanten elektronischen Komponenten des Injektionsgeräts. Mit Ausnahme des Produktbehälters und von metallischen Komponenten wie mechanischen Federn oder elektrischen Leitern in Form von Drähten oder flexiblen Leiterbahnen verbleiben also nach dem Trennvorgang nur Kunststoffteile des Injektionsgeräts, welche auf bekanntem Wege dem medizinischen Abfall zugeführt werden können.

Erfindungsgemäss umfasst das Injektionsgerät einen mechanischen Sicherungsmechanismus zur Verhinderung oder Sperrung des Trennvorgangs vor Beginn der Ausschüttung und zur Freigabe oder Entsperrung des Trennvorgangs frühestens mit oder nach der Auslösung der automatischen Ausschüttung. Dadurch wird verhindert, dass das Elektronikmodul vor Beginn der Injektion vom Injektionsgerät entfernt wird und der Detektor den Beginn der Injektion nicht detektieren kann. Die Freigabe des Trennvorgangs erfolgt spätestens mit dem letzten Schritt des Injektionsvorganges, und bevorzugt vor der Entfernung des Injektionsgeräts von einer Einstichstelle. Dadurch wird zusätzlich sichergestellt, dass der Trennvorgang ausgeführt und das Elektronikmodul vom Injektionsgerät getrennt werden kann, auch wenn beim Entfernen des Injektionsgeräts von der Einstichstelle eine Fehlmanipulation oder Probleme mit der Nadelschutzvorrichtung auftreten sollten.

In einer vorteilhaften Variante umfasst der Trennvorgang eine drehende Lösebewegung des Modulgehäuses gegenüber dem Gerätegehäuse, auf welche ähnlich einem Bajonettverschluss noch eine axiale Lösebewegung folgen kann, oder welcher ähnlich einem kindersicheren Sicherheitsdrehverschluss eine axiale Lösebewegung des Modulgehäuses auf das Gerätegehäuse zu vorhergehen kann. Der Trennvorgang umfasst alternativ eine rein axiale Lösebewegung des Modulgehäuses von dem Gerätegehäuse weg, beispielsweise zur Lösung einer Schnappverbindung zwischen Modul- und Gerätegehäuse.

Im Gegensatz zu einer rein rotierenden oder transversalen Lösebewegung ist insbesondere eine axiale Lösebewegung des Modulgehäuses auf das Gerätegehäuse zu mit einem Kraftaufwand verbunden, welcher eine für den Anwender bei aufgesetztem Injektionsgerät schmerzhafte Bewegung der Nadel in der Injektionsstelle verursacht. Der Anwender wird durch die Erwartung dieser Schmerzen zusätzlich motiviert mit der axialen Lösebewegung bis nach dem Entfernen des Injektionsgeräts von der Injektionsstelle zuzuwarten. Ein notwendiger Einsatz eines Werkzeuges zur Ausführung des Trennvorgangs des zur Trennung freigegebenen Modulgehäuses von dem Gerätegehäuse kann den Anwender weiter motivieren mit der Trennung des Elektronikmoduls vom Injektionsgerät bis nach der Injektion zuzuwarten.

In einer bevorzugten Ausführungsform umfasst der mechanische Sicherungsmechanismus ein Freigabeelement in Form eines Bügels, einer Lasche, oder eines Riegels; entweder als eigenständiges Bauteil, oder als Teil eines gehäusefest angebrachten Gehäuseeinsatzes, oder einstückig ausgebildet mit dem Modul- oder Gerätegehäuse. Das Freigabeelement sperrt oder blockiert in einer Sperrstellung des Freigabeelements die Lösebewegung vor Beginn der Ausschüttung, und gibt die Lösebewegung frei mit oder nach der Auslösung der Ausschüttung. Das Freigabeelement nimmt dazu vor dem Entfernen des Injektionsgeräts von der Injektionsstelle eine Freigabestellung ein, bewirkt unmittelbar durch eine Auslösebewegung des Anwenders, beispielsweise eine Verschiebung der Nadelschutzhülse bei Aufdrücken des Gerätes auf die Injektionsstelle, oder durch eine Ausschüttfeder des Antriebs nach Auslösung, oder durch eine Signalisierungsfeder zur akustischen oder taktilen Signalisierung eines Beginns oder Endes der Ausschüttung.

Die Einnahme der Freigabestellung erfolgt also vor dem Entfernen des Injektionsgerätes von der Injektionsstelle, und wird nicht durch eine bei dieser Gelegenheit nach distal expandierende Nadelschutzfeder bewirkt. Der Freigabehub des Freigabeelements in die Freigabestellung erfolgt bevorzugt gegen eine Vorspannung des Freigabeelements in die Sperrstellung, mit welcher eine spontane Freigabe des Trennvorgangs verhindert wird, wobei Letzteres auch durch axiale Reibkräfte am Freigabeelement geschehen kann. Die Bewegung des Freigabeelements in die Freigabestellung kann an Stelle einer unmittelbaren Wirkung auch erst ein zusätzliches Sperrelement in eine die Lösebewegung ermöglichende Freigabeposition drängen.

In einer bevorzugten Ausführungsform umfasst das Injektionsgerät eine Ausschüttfeder in Form einer Druckfeder zum Antrieb eines Vortriebselements in Form einer Kolbenhülse, sowie ein Halteelement zum Halten der Ausschüttfeder in einem vorgespannten Zustand vor der Auslösung, an dessen proximalen Ende sich die Druckfeder abstützt. Das Freigabeelement wird in die Freigabestellung bewegt durch eine proximale Bewegung des Halteelements, verursacht durch die Ausschüttfeder zu Beginn oder am Ende der Ausschüttung. Alternativ umfasst das Injektionsgerät eine Ausschüttfeder in Form einer Torsionsfeder und ein rotierendes Antriebselement in Form einer Gewindestange zum Antrieb eines Vortriebselements in Form einer Kolbenhülse mit Innengewinde. Das Freigabeelement wird in die Freigabestellung bewegt durch eine proximale Bewegung Antriebselements beim Aufbau eines Gegendrucks auf das Vortriebselement durch die Flüssigkeit im Produktbehälter.

In einer bevorzugten Ausführungsform umfasst der Trennvorgang eine axiale Lösebewegung in Richtung einer Längsachse des Injektionsgeräts, wobei das Modulgehäuse durch Schnappelemente am oder zum Gerätegehäuse oder einem Gehäuseeinsatz verschnappt ist, welche von dem Freigabeelement in der Sperrstellung über nicht-axiale Sperrflächen am Lösen oder Ausschnappen gehindert werden.

In einer bevorzugten Ausführungsform umfasst der Trennvorgang eine rotierende Lösebewegung in einer Ebene senkrecht zu einer Längsachse des Injektionsgeräts. Dabei wird ein radial nach innen weisender Vorsprung am Modulgehäuse vom Freigabeelement in der Sperrstellung über nicht-tangentiale Sperrflächen am Rotieren gehindert. Alternativ, falls das Freigabeelement selbst bei der Lösebewegung mitrotiert, wird letzteres in der Sperrstellung durch einen radial nach innen weisenden Vorsprung am Gerätegehäuse oder an einem Gehäuseeinsatz im Gerätegehäuse am Rotieren gehindert.

In einer bevorzugten Ausführungsform umfasst das Elektronikmodul eine Kommunikationseinheit oder einen Sender, und eine Batterie oder einen sonstigen Energiespeicher zur Speisung der Kommunikationseinheit, welche in einem Batteriefach gehalten ist und erst bei abgetrenntem Modulgehäuse vom Elektronikmodul entfernt und einer spezifischen Sammelstelle zugeführt werden kann. Bevorzugt ist die Batterie im Batteriefach nur leicht oder gar nicht geklemmt und fällt bei oder nach der Trennung rein auf Grund der Schwerkraft aus dem Batteriefach, wobei sie dabei noch an einem nicht-reissfesten Faden gehalten werden kann und somit nicht verloren geht.

In einer bevorzugten Ausführungsform ist der Sensor ausgebildet und/oder angeordnet zur Detektion einer durch die Ausschüttfeder bewirkten axialen oder radialen Bewegung des Halteelementes, des Antriebselements, oder des Vortriebselements. Insbesondere die Bewegungen des Halteelements und des Antriebselements können dabei in proximale Richtung entgegen der Ausschüttrichtung erfolgen und nach einem kurzen Weg von einem gehäusefesten Abstütz- oder Anschlagelement abgefangen werden. Alternativ ist der Sensor ausgebildet zur Detektion einer durch eine Nadelschutzfeder bewirkten axialen Bewegung einer Nadelschutzhülse oder eines Signalglieds, insbesondere der An- oder Abwesenheit des Signalglieds in seiner proximalen Position in Kontakt mit einem Signalanschlag. Ein einziger Sensor kann sowohl den Start wie auch das Ende der Ausschüttung feststellen, beispielsweise falls das Signalglied zu Beginn der Ausschüttung durch die Kraft der Ausschüttfeder und unter Spannung der Nadelschutzfeder um einen Spann-hub von dem Signalanschlag weg bewegt wird. Ansonsten können auch zwei oder mehrere Sensoren vorgesehen sein. Eine axiale Verlängerung in Form eines Stössels oder Pins an den besagten beweglichen Elementen und/oder am Sensor, oder durch einen separaten Schaltadapter realisiert, kann den mechanischen Kontakt oder gegenseitigen Kraftschluss zum Sensor sicherstellen.

Das Elektronikmodul umfasst eine Prozessoreinheit, welche durch einen mechanischen Schaltdetektor als Sensor aus einem stromlosen Zustand eingeschaltet oder durch einen elektronischen Detektor als Sensor aus einem energiesparenden Modus geweckt wird. Die detektierte Ausschüttung, zusammen mit einem Zeitstempel und weiteren Angaben wie der Dauer der Ausschüttung oder einer Haltezeit, werden durch die Kommunikationseinheit drahtlos an ein Mobilgerät übermittelt. Diese Übermittlung kann auch nach Abtrennung des Elektronikmoduls geschehen oder beendet werden. Die Prozessoreinheit kann zusätzlich konfiguriert sein zur Erzeugung eines akustischen oder taktilen Hinweises an den Anwender, welcher den Ablauf einer Haltezeit von einigen wenigen Sekunden nach Ende der Ausschüttung anzeigt. Ein beispielhaftes visuelles Feedback zum Ablauf der Haltezeit umfasst den Wechsel einer LED von einem blinkenden zu einem ruhenden Zustand oder umgekehrt, dieser Hinweis stoppt spätestens mit dem Abheben des Injektionsgeräts von der Injektionsstelle.

Bevorzugt ist das Injektionsgerät ein Autoinjektor mit einer bei Lieferung beziehungsweise vor Inbetriebnahme des Autoinjektors maximal vorgespannten Ausschüttfeder für eine einmalige Ausschüttung des gesamten oder zumindest eines vorbestimmten Inhalts des Produktbehälters. Der Autoinjektor verfügt dementsprechend nicht über einen Dosiswahlmechanismus. Eine vorgefüllte Einweg-Fertigspritze umfasst den Produktbehälter und eine daran unlösbar befestigte Injektionsnadel und ist axial fixiert im Gehäuse des Autoinjektors gehalten. Der Autoinjektor, oder zumindest die Fertigspritze und der Spritzenhalter, sind entsprechend nur für einen einmaligen Gebrauch vorgesehen.

Das Elektronikmodul kann dabei neben der Prozessoreinheit zur Auswertung der Daten des Sensors eine Bluetooth-Sendevorrichtung zur drahtlosen Übertragung von Information der Ausschüttung umfassen. Die Sendevorrichtung ist ausgebildet zur wiederholten Übertragung der bevorzugt geeignet verschlüsselten Information zusammen mit einer nicht-verschlüsselten, eindeutigen Kennung des Autoinjektors. Die Sendevorrichtung operiert bevorzugt und exklusiv als Beacon in einem Bluetooth Broadcast-Modus und kommuniziert die Informationen und die Kennung in Form von Advertising-Paketen.

Ein weiterer Aspekt der Erfindung betrifft den modularen Aufbau einer Gerätefamilie oder Plattform mit zwei Autoinjektoren, welche möglichst viele identische oder austauschbare Komponenten aufweisen und von welchen nur ein erster Autoinjektor über ein Elektronikmodul verfügt. Eine erfindungsgemässe Gerätefamilie umfasst also einen ersten und einen zweiten Autoinjektoren, beide mit demselben Gerätegehäuse, demselben Spritzenhalter zur Aufnahme einer Fertigspritze, demselben Vortriebselement zum Ausschütten einer Dosis durch Vortrieb eines Kolbens in der Spritze, demselben Auslösemechanismus zum Auslösen der Ausschüttung, und derselben Nadelschutzhülse. Der erste Autoinjektor weist am proximalen Ende ein Modulgehäuse auf zur Abdeckung einer Öffnung des Gerätegehäuses, welches ein Elektronikmodul aufnimmt und vom Gerätegehäuse gelöst werden kann. Der zweite Autoinjektor weist am proximalen Ende eine End- oder Abschlusskappe auf zur Abdeckung einer Öffnung des Gerätegehäuses, welche dauerhaft und unlösbar beziehungsweise nicht zerstörungsfrei mit dem Gerätegehäuse verbunden, bevorzugt verschnappt, verschweisst, oder verleimt ist.

Bevorzugt sind auch die Nadelschutzhülsenfedern zur Bewegung der Nadelschutzhülse in eine Nadelschutzposition sowie die Ausschüttfedern der beiden Autoinjektoren identisch. Allerdings kann der fehlende Antrieb eines Freigabeelements beim zweiten Autoinjektor auch eine mit weniger Marge dimensionierte Feder akzeptieren.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden. Es zeigen
- Fig. 1: eine Explosionsdarstellung eines Autoinjektors mit Druckfederantrieb;
- Fig.2: eine erste Ausführungsform des Sicherungsmechanismus;
- Fig.3: eine erste Variante einer zweiten Ausführungsform des Sicherungsmechanismus;
- Fig.4: eine zweite Variante der zweiten Ausführungsform;
- Fig.5: eine dritte Variante der zweiten Ausführungsform;
- Fig.6: einen partiellen Längsschnitt eines Autoinjektors mit Torsionsfederantrieb; und
- Fig.7: eine dritte Ausführungsform des Sicherungsmechanismus.

### FIGURENBESCHREIBUNG

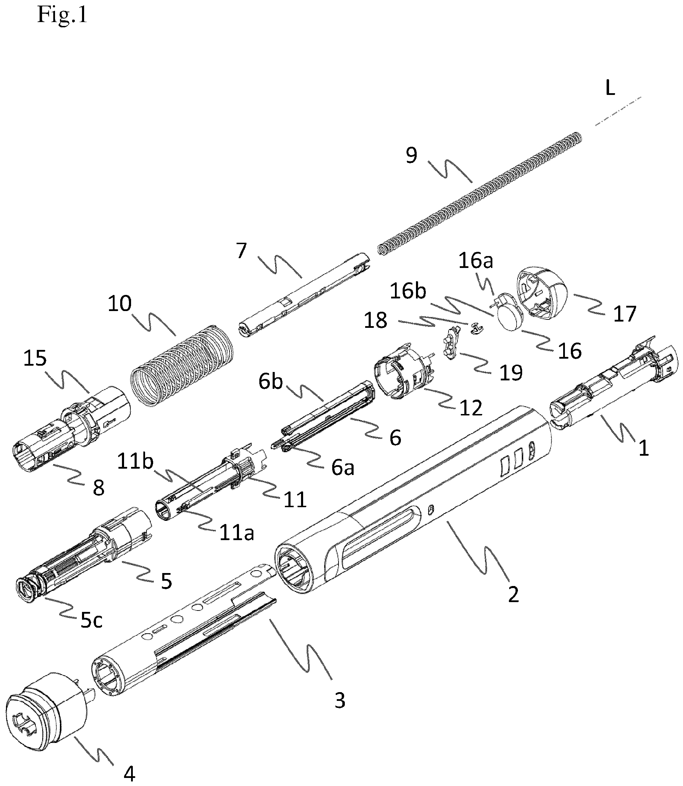

Fig.1 zeigt eine Explosionsdarstellung eines Autoinjektors umfassend einen Antrieb mit einer als Wendelfeder ausgebildeten Druckfeder. Der Autoinjektor weist ein hülsenförmiges, längliches Injektor- oder Gerätegehäuse 2 mit einer Längsachse L auf, welches von einem Anwender während der Injektion in einer Hand gehalten wird. Eine nicht dargestellte Fertigspritze ist in einem Spritzenhalter 1 aufgenommen, welcher wiederum im Gehäuse 2 dreh- und axialfest verschnappt ist. An dem distalen Ende des Gehäuses 2 ist im Auslieferungszustand eine Abziehkappe 4 angeordnet, die vor der Verwendung des Autoinjektors entfernt wird. Eine Nadelschutzhülse 3 ist relativ zu dem Gehäuse 2 und entlang der Längsachse L um einen Betätigungshub H_{B} in die proximale Richtung in eine betätigte Position verschiebbar um eine Produktausschüttung auszulösen. Ein Mechanikhalter 5 ist dreh- und axialfest mit dem Gehäuse 2 verschnappt und drückt mittels eins Haltefederabschnitts 5c die Fertigspritze nach distal in den Spritzenhalter 1.

Der Autoinjektor weist eine Ausschüttfeder 9 in Gestalt einer als Druckfeder wirkenden Wendelfeder auf, welche im Auslieferungszustand annähernd vollständig von einem hülsenförmigen Vortriebsglied 7 umgeben ist und auf dieses eine Kraft in distaler Richtung ausübt. Die Ausschüttfeder 9 stützt sich mit ihrem proximalen Ende an einem Halteelement 6 ab, welches zwei Arme 6b aufweist, wobei an jedem Arm 6c ein Eingriffselement 6a angeordnet ist. Die Eingriffselemente 6a weisen radial zu der Längsachse L hin und greifen im Auslieferungszustand in Ausnehmungen am auf der Aussenfläche des Vortriebselements 7 ein. Ein Schaltmodul mit einer Schalthülse 15 und einer Sperrhülse 8 verhindert ein Auslenken der zwei Arme 6b, wodurch auch eine Bewegung des Vortriebsglieds 7 relativ zu dem Halteelement 6 in die distale Richtung verhindert wird. Die Schalthülse 15 ist zumindest kraftschlüssig mit dem proximalen Ende der Nadelschutzhülse 3 verbunden und wird von einer Nadelschutzfeder 10 nach distal gedrängt. Zur Auslösung der Ausschüttung werden die Nadelschutzhülse 3 und das Schaltmodul 15, 8 nach proximal bewegt, wodurch die Nadelschutzfeder 10 gespannt wird und die Arme 6b auslenken können. Dadurch ist die axialfeste Kopplung zwischen dem Vortriebsglied 7 und dem Halteelement 6 aufgehoben, und während Ersteres nach distal bewegt wird kann sich das Halteelement 6 um einen kleinen Initialhub bis zu einem axialfesten Anschlag nach proximal verschieben. Bei Entfernung des Autoinjektors von der Injektionsstelle wird die Nadelschutzhülse 3 durch die Nadelschutzfeder 10 nach distal bewegt und vom Schaltmodul 15, 8 in einer Nadelschutzposition verriegelt.

Die Nadelschutzfeder 10 ist eine als Druckfeder wirkende und als Wendelfeder ausgestaltete Feder aus Metall und stützt sich mit ihrem proximalen Ende an einem Signalglied 11 ab. Das Signalglied 11 ist im Auslieferungszustand in Kontakt mit einem Signalanschlag eines Gehäuseeinsatzes 12, und weist zwei Arme 11b auf, wobei an jedem Arm 11b ein Eingriffselement 11a angeordnet ist. Die Eingriffselemente 11a weisen radial zu der Längsachse L hin und greifen im Auslieferungszustand in weitere Ausnehmungen des Vortriebselements 7 ein. Zu Beginn der Ausschüttung wird das Signalglied 11 dadurch um einen Spann-hub von dem Signalanschlag 12a weg bewegt, anschliessend durch die Aussenfläche des Vortriebsglieds in einem Eingriff mit der Sperrhülse 8 gehalten, und am Ende der Ausschüttung für eine durch die Nadelschutzfeder 10 beschleunigte Bewegung nach proximal auf den Signalanschlag 12a zu freigegeben. Details zur Ausgestaltung des Autoinjektors mit Druckfederantrieb sind beschrieben in der Patentanmeldung PCTEP2021052898.

Der Gehäuseeinsatz 12 ist formschlüssig dreh- und axialfest mit dem proximalen Ende des Gehäuses 2 verbunden und kann dieses auch axial ergänzen oder verlängern und allgemein als Moduladapter für ein Elektronikmodulgehäuse oder Geräteendkappe dienen. Am proximalen Ende des Gehäuseeinsatzes 12 ist ein lösbares Modulgehäuse 17 für ein Elektronikmodul 16 vorgesehen. Das Elektronikmodul 16 umfasst einen Sensor 16a und eine Batterie 16b, weiter eine Prozessoreinheit, eine Kommunikationseinheit, und/oder eine Lichtquelle zur optischen Signalisierung eines Betriebszustandes. Der Sensor 16a ist ausgebildet zur Detektion oder Überwachung der Anwesenheit des Signalglieds 11 in seiner proximalen Position in Kontakt mit dem Signalanschlag 12a, beziehungsweise der Abwesenheit des Signalglieds von dieser Position während der Dauer der Ausschüttung. Der Sensor 16a des Elektronikmoduls 16 ist als Schaltdetektor ausgebildet, der Sensor 16a und/oder das Signalglied 11 weisen einen axialen Ansatz in Form eines Stössels auf um einen gegenseitigen Kraftschluss sicherzustellen. Alternativ kann ein Schaltadapter als separates Bauteil vorgesehen sein zur Übertragung der Position des Signalglieds 11 zum Schaltdetektor über eine Axial-, Kipp-, oder sonstige Umlenkbewegung. Ein Prozessor des Elektronikmoduls 16 wertet die detektierten Bewegungen des Signalglieds aus und meldet insbesondere den Zeitpunkt einer erfolgten Injektion über ein Kommunikationsmodul.

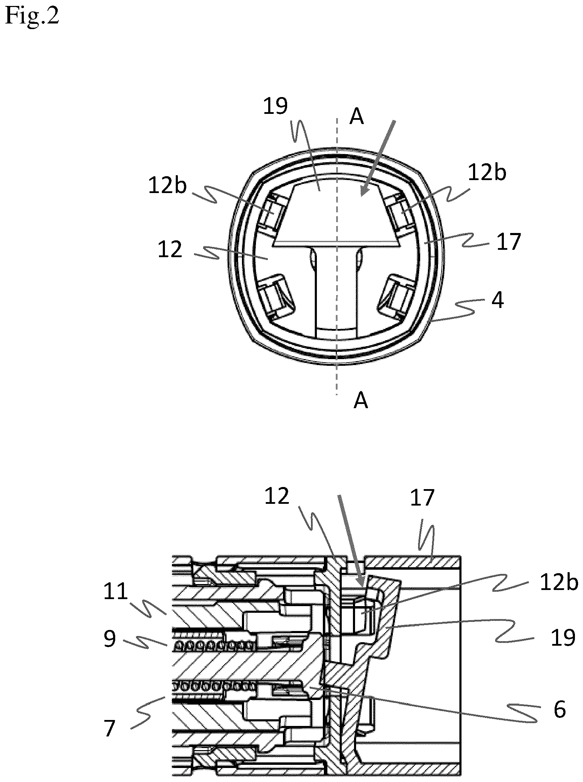

Fig.2 zeigt eine Axialsicht des Autoinjektors von hinten bei abgeschnittenem Modulgehäuse 17 und ohne Elektronikmodul (oben) sowie einen Ausschnitt eines Längsschnitt durch eine Ebene A-A (unten). In dieser Ausführungsform der Erfindung ist das Modulgehäuse 17 mittels einer Schnappverbindung am Gehäuseeinsatz 12 befestigt. Zwei flexible Schnappelemente 12b am Gehäuseeinsatz hintergreifen radial nach innen vorstehende Abragungen des Modulgehäuses 17 und werden durch ein Freigabeelement 19 in Form eines Biegearms am Modulgehäuse von einer Auslenkung nach innen gehindert. Durch den kleinen Initialhub des Halteelements 6 nach proximal wird das Freigabeelement 19 ausgelenkt beziehungsweise verbogen und die Schnappelemente 12b freigegeben, so dass nun das Modulgehäuse 17 durch den Anwender axial nach hinten vom Gehäuseeinsatz entfernt werden kann.

Die Funktionen und Eigenschaften des Gehäuseeinsatzes können auch direkt vom Gehäuse des Injektionsgerätes übernommen werden, ein separater aber fest verbundener Gehäuseeinsatz ist primär aus fertigungstechnischen Gründen vorteilhaft. Dementsprechend können die Schnappelemente auch am Gehäuse des Injektionsgerätes vorgesehen sein, oder auch am Modulgehäuse angebracht werden und in Öffnungen des Gehäuses einschnappen. Zudem kann bei diesem Konzept das Abtrennen des Modulgehäuses mittels Schraubendreher oder ähnlichem Werkzeug geschehen. Dazu wird der Schraubendreher in Pfeilrichtung durch eine Öffnung im Modulgehäuse unter ein elastisches Ende des Freigabeelements geführt und verkippt. Das Freigabeelement kann auch ein eigenes Bauteil sein und durch das Halteelement gesamthaft nach hinten bewegt werden. Dadurch wird das Freigabeelement nicht verbogen und kann auch diagonal einander gegenüberliegende Schnappelemente freigeben.

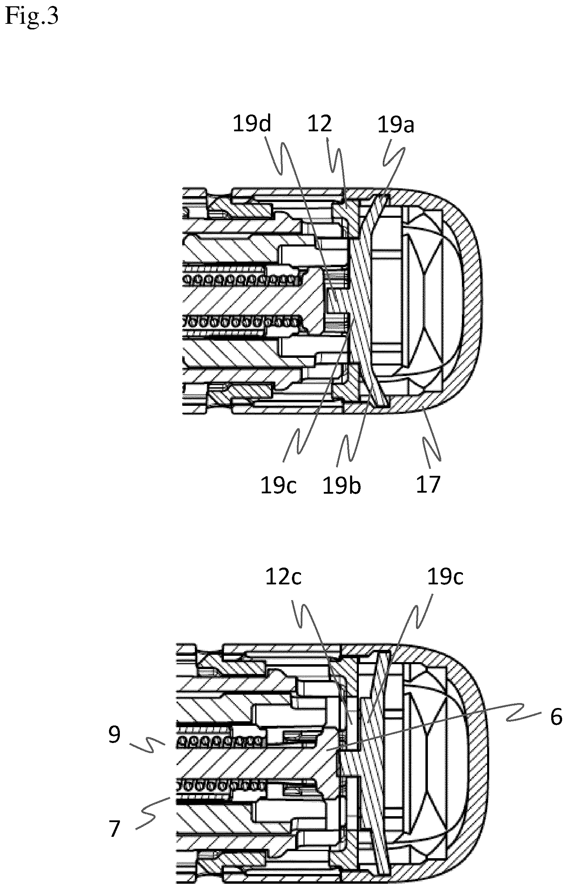

Fig.3 zeigt einen Längsschnitt einer ersten Variante einer zweiten Ausführungsform der Erfindung, bei welcher das Modulgehäuse mittels Drehbewegung gelöst werden kann. Ein Freigabeelement 19 ist über zwei einander diagonal gegenüberliegende Verankerungen 19a drehfest im Modulgehäuse 17 verankert und über zwei Federarme 19b gegen distal vorgespannt, so dass im Sperrzustand (oben) ein abgerundet rechteckiges Sperrprofil 19c des Freigabeelements 19 in eine passende Profilaufnahme 12c des Gehäuseeinsatzes 12 eingreift. Eine stiftförmige distale Fortsetzung 19d des Freigabeelements trifft auf das proximale Ende des Halteelements 6 und wird durch dessen Initialhub gegen die Vorspannung der Federarme 19b nach proximal gedrückt, wodurch das Sperrprofil 19c aus der Aussparung 12c bewegt wird und das Freigabeelement 19 mit dem Modulgehäuse 17 rotiert werden kann (unten).

An Stelle des rechteckigen Sperrprofils kann ein beliebiges um die Längsachse L nichtrotationssymmetrisches Profil treten zur Aufnahme eines Drehmoments, beispielsweise eine Kreuz- oder eine Sternform, oder ein Profil mit mehreren Ausprägungen zum Eingriff in mehrere Einprägungen. Das Sperrprofil kann eine distal vorstehende Struktur am Freigabeelement umfassen zum Eingriff in eine Aussparung am Gehäuseeinsatz, oder eine nach proximal vorstehende Ausbuchtung am Gehäuseeinsatz zum Eingriff in eine korrespondierende Einbuchtung am Freigabeelement. An Stelle der Federarme können separate Federelemente zur distalen Vorspannung des Freigabeelementes vorgesehen sein und so eine spontane Freigabe der Lösebewegung verhindern, oder das Freigabeelement ist über die stiftförmige distale Fortsetzung kraftschlüssig mit dem Halteelement verbunden. Auf die drehende Lösebewegung des Modulgehäuses gegenüber dem Gerätegehäuse kann ähnlich einem Bajonettverschluss noch eine axiale Lösebewegung folgen, oder die Drehbewegung ist Teil einer schraubenden Lösebewegung.

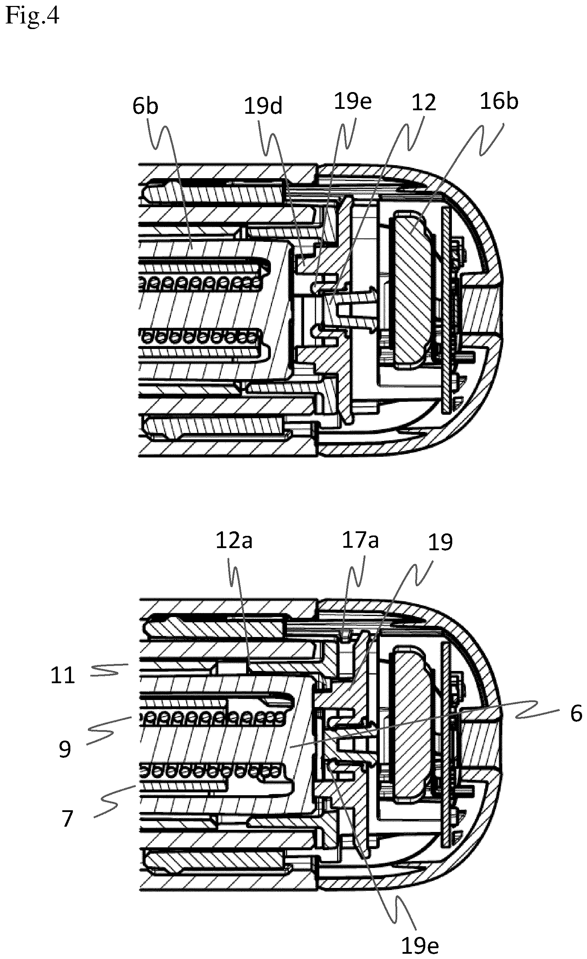

Fig.4 zeigt einen Längsschnitt einer zweiten Variante der zweiten Ausführungsform der Erfindung im Auslieferungszustand (oben) und im ausgelösten Zustand (unten). Die Schnittebene ist gegenüber derjenigen in Fig.3 um 90° gedreht, dadurch sind neben einem zentralen Pin auch die Arme 6b des Halteelements 6 ersichtlich. Das Freigabeelement 19 ist im Auslieferungszustand mittels Schnappern 19e gehalten am Gehäuseeinsatz 12. Die Schnapper 19e ersetzen die Federarme der ersten Variante und vermögen zumindest das Eigengewicht des Freigabeelements 19 gegen eine Bewegung nach proximal in eine Freigabestellung des Freigabeelementes zu halten. Nach der Auslösung drückt die Ausschüttfeder 9 via die Fortsetzungen 19d das Freigabeelement 19 nach proximal und damit die Schnapper 19e aus ihrer Halteposition. Gegenüberliegende periphere Enden des Freigabeelements werden axial von Nocken 17a wegbewegt und stellen diese für eine Drehbewegung frei. Alternativ oder komplementär dazu ist wie in der vorhergehenden Variante eine Rotationsblockade mittels Profilaufnahme und Sperrprofil möglich.

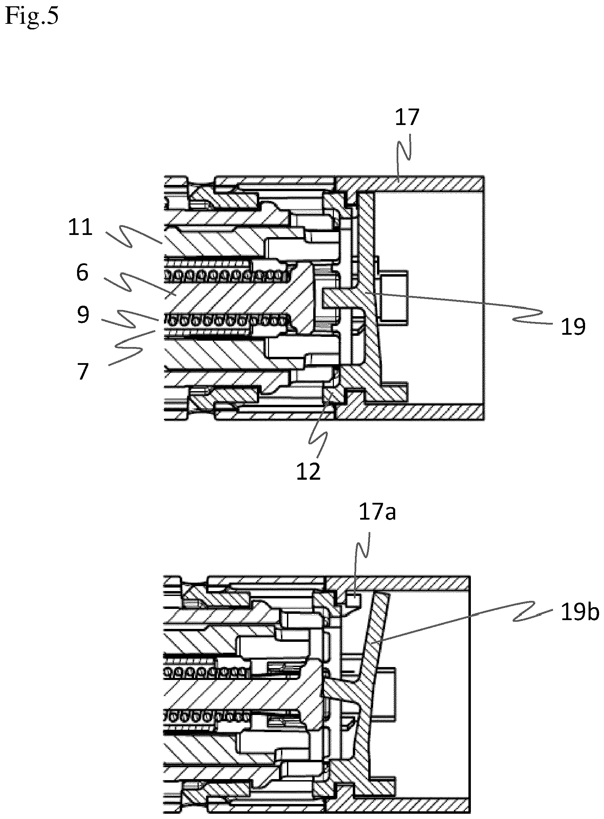

Fig.5 zeigt einen Längsschnitt einer dritten Variante der zweiten Ausführungsform der Erfindung im Auslieferungszustand (oben) und im ausgelösten Zustand (unten), bei welcher das Freigabeelement 19 einstückig mit dem Gehäuseeinsatz 12 ausgebildet ist und somit bei der drehenden Lösebewegung selbst nicht rotiert. Das Freigabeelement 19 ist also kein separates Bauteil und kann somit auch nicht im Moment des Entfernens des Modulgehäuses 17 herausfallen und verloren gehen. Wie in der ersten Ausführungsform ist das Freigabeelement 19 elastisch oder gelenkig flexibel und einseitig mit dem Gehäuseeinsatz verbunden. Das gegenüberliegende Ende des Federarmes 19b ist in einem rotationsblockierenden Anschlag mit einem radial nach innen vorstehenden Nocken 17a des Modulgehäuses 17. Durch den Initialhub des Halteelementes 6 wird das Ende des Federarms 19b nach proximal angehoben und der Nocken 17a zur Drehung freigegeben.

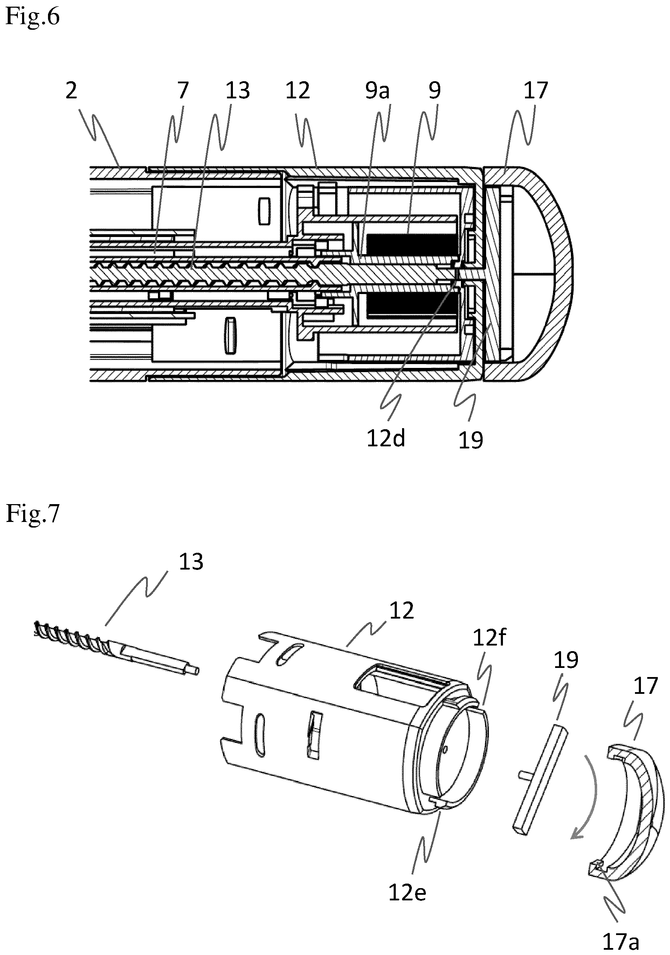

Fig.6 zeigt einen Längsschnitt des proximalen Endes eines Autoinjektors umfassend einen Antrieb mit einer als Spiralfeder ausgebildeten Torsionsfeder und ansonsten grösstenteils funktionsgleichen Komponenten wie in der zweiten Ausführungsform. Der Autoinjektor umfasst ein Gehäuse 2, einen mit dem Gehäuse fest verbundenen Gehäuseeinsatz 12, ein Antriebselement 13 in Form einer Gewindestange, ein Vortriebselement 7 in Form einer Hülse mit einem Innengewinde in Eingriff mit dem Gewinde der Gewindestange, und eine Ausschüttfeder 9 in Form einer Trieb- oder Spiralfeder. Letztere ist auf dem Schaft einer Federspule 9a aufgewickelt und mit einem inneren Ende rotationsfest mit diesem verbunden. Die Federspule 9a wiederum ist rotationsfest aber nicht axialfest mit dem Antriebselement 13 gekoppelt. Eine Nadelschutzhülse dient auch als Auslöseelement zum Auslösen der Produktausschüttung, wobei ein axial mit der Nadelschutzhülse gekoppeltes Schaltmodul ein Halteelement freigibt und dadurch der axiale Vortrieb des Vortriebselements 7 oder die Rotation des Antriebselements 13 oder der Federspule 9a ermöglicht. Nach Auslösung der Ausschüttung wird das Vortriebselement 7 in Anschlag gebracht mit dem Stopfen der Spritze, worauf sich in der Flüssigkeit der Spritze ein Gegendruck auf den Stopfen aufbaut, das längsgeführte Vortriebselement 7 kurz blockiert und sich dadurch die drehende Gewindestange als Antriebselement 13 nach proximal schraubt. Sobald die Gewindestange ihrerseits mit einer Schulter an einem nach distal gerichteten Anschlag 12d ansteht wird der Gegendruck überwunden und das Vortriebselement 7 vollendet den Ausschütthub. Die Proximalbewegung des Antriebselements 13 ist vergleichbar mit dem Initialhub des Halteelements der Druckfeder-Ausführungsformen und ausreichend für eine axiale Lösebewegung des Freigabeelements 19. Details zur Ausgestaltung des Autoinjektors mit Torsionsfederantrieb sowie eines Rotationssensors zur abwechslungsweisen kontinuierlichen Detektion von mindestens zwei Rotationspositionen pro Umdrehung des Antriebselementes während der Ausschüttung sind in der Patentanmeldung PCTEP2021057168 offenbart.

Fig.7 zeigt eine Explosionsansicht von vier Komponenten der Ausführungsform aus Fig.6 mit einem Bajonettverschluss zwischen Modulgehäuse 17 und Gehäuseeinsatz 12. Das Modulgehäuse 17 ist zusätzlich entlang der Längsachse geschnitten. Die Trennbewegung umfasst also eine Drehung des Modulgehäuses relativ zum Gehäuseeinsatz 12 im durch den Pfeil angegebenen Drehsinn. Dazu weist der Gehäuseeinsatz 12 auf einer Aussenseite eine Drehführung 12e für einen Nocken 17a auf der Innenseite des Modulgehäuses 17 auf. Die Ausgangsposition des Nockens 17a in der Drehführung 12e wird durch eine schlitzförmige Axialführung 12f begrenzt, durch welche ein Ende des Freigabeelements 19 ragt. Letzteres ist mit einem proximalen Ende des Antriebselements 13 axial gekoppelt und wird durch die Proximalbewegung des Antriebselements 13 um mindestens die axiale Ausdehnung des Nockens 17a angehoben. Das dargestellte Modulgehäuse umfasst zwei Nocken 17a mit je einer Winkelausdehnung von ungefähr 45°, wobei mit einer Winkelausdehnung von weniger als 45° bis zu vier Drehführung/Nockenpaare möglich sind.

Als Alternative zu der beschriebenen Proximalbewegung des Antriebselements kann eine Drehbewegung der Gewindestange über einen Gewindeeingriff mit dem Freigabeelement in eine axiale Bewegung des Freigabeelements umgesetzt werden. Dazu verfügen die Gewindestange und das Freigabeelement über einen Gewindeüberlapp entsprechend dem Freigabehub des Freigabeelements, so dass das Freigabeelement am Ende der Proximalbewegung aus dem Eingriff mit der Gewindestange gelangt und von dieser axial wegbewegt werden kann. Ebenfalls denkbar ist ein einseitig wirkendes flexibles Freigabeelement wie in Fig.5, welches einseitig mit dem Gehäuseeinsatz 12 oder dem Modulgehäuse 17 fest verbunden ist.

Weiter alternativ kann die Proximalbewegung des Freigabeelements durch einen Finalhub des Halteelements oder eines Signalisierungselements wie oben beschrieben am Ende der Ausschüttung verursacht werden. Insbesondere geeignet ist jedoch ein Signalisierungselement ohne Initial- oder Ladebewegung, d.h. mit ausschliesslich einer Signalisierungsbewegung gegen einen Anschlag am proximalen Ende des Autoinjektors, also beispielsweise ein Signalisierungselement an welchem sich die Ausschüttfeder gegen proximal abstützt, und welches am Ende der Ausschüttung vom proximalen Ende des Vortriebsglied für eine Signalisierungsbewegung nach proximal freigegeben wird.

**BEZUGSZEICHENLISTE**

| | | | |
|---|---|---|---|
| 1 | Spritzenhalter | 12c | Aussparung |
| 2 | Gehäuse | 12d | Anschlag |
| 3 | Nadelschutzhülse | 12e | Drehführung |
| 4 | Abziehkappe | 12f | Axialführung |
| 5 | Mechanikhalter | 13 | Antriebselement |
| 5c | Haltefederabschnitt | 14 | Nadelschutzkappe |
| 6 | Halteelement | 15 | Schalthülse |
| 6a | erstes Eingriffselement | 16 | Elektronikmodul |
| 6b | Arm | 16a | Sensor |
| 7 | Vortriebsglied | 16b | Batterie |
| 8 | Sperrhülse | 17 | Modulgehäuse |
| 9 | Ausschüttfeder | 17a | Nocken |
| 9a | Federspule | 18 | Schaltadapter |
| 10 | Nadelschutzfeder | 19 | Freigabeelement |
| 11 | Signalglied | 19a | Verankerung |
| 11a | erstes Eingriffsglied | 19b | Federarm |
| 11b | Arm | 19c | Sperrprofil |
| 12 | Gehäuseeinsatz | 19d | Fortsetzung |
| 12a | Signalanschlag | 19e | Schnapper |
| 12b | Schnappelement | L | Längsachse |

## Patentansprüche

1. Injektionsgerät zur subkutanen Verabreichung eines maximalen Inhalts eines Produktbehälters durch eine Kanüle an einem distalen Ende des Produktbehälters, umfassend
- ein Gerätegehäuse (2) mit einer Öffnung an einem proximalen Ende,
- ein Modulgehäuse (17) zur Abdeckung der Öffnung, welches von dem Gerätegehäuse (2) durch einen Trennvorgang trennbar ist,
- ein in dem Gerätegehäuse (2) angeordneter Antrieb (9, 13) zur Bewegung eines Vortriebsglieds (7) und zur einmaligen automatischen Ausschüttung des Inhalts des Produktbehälters,
- ein in dem Modulgehäuse (17) gehaltenes Elektronikmodul (16) mit einem Sensor (16a) zur Detektion der Ausschüttung,
**gekennzeichnet durch** einen mechanischen Sicherungsmechanismus (19, 12b; 12c, 19c; 17a) zur Freigabe des Trennvorgangs mit oder nach der Auslösung der automatischen Ausschüttung.

2. Injektionsgerät nach Anspruch 1, **dadurch gekennzeichnet, dass** der Trennvorgang eine drehende Lösebewegung des Modulgehäuses (17) gegenüber dem Gerätegehäuse (2), und/oder eine axiale Lösebewegung des Modulgehäuses (17) auf das Gerätegehäuse (2) zu, und/oder eine axiale Lösebewegung des Modulgehäuses (17) von dem Gerätegehäuse (2) weg umfasst.

3. Injektionsgerät nach Anspruch 2, **dadurch gekennzeichnet dass** der mechanische Sicherungsmechanismus ein Freigabeelement (19) umfasst zur Sperrung der drehenden oder axialen Lösebewegung vor der Auslösung der Ausschüttung in einer Sperrstellung des Freigabeelements (19) und zur Freigabe der Lösebewegung vor dem Entfernen des Injektionsgeräts von einer Injektionsstelle in einer Freigabestellung des Freigabeelements (19).

4. Injektionsgerät nach Anspruch 3, wobei das Injektionsgerät eine Ausschüttfeder (9) zum Antrieb eines Vortriebselements (7) sowie ein Halteelement (6) zum Halten der Ausschüttfeder (9) in einem vorgespannten Zustand vor der Auslösung oder ein Antriebselement (13) zur Übertragung einer Ausschüttkraft der Ausschüttfeder (9) auf das Vortriebselement (7) umfasst, **dadurch gekennzeichnet, dass** das Freigabeelement (19) in die Freigabestellung bewegt wird durch eine proximale Bewegung des Halteelements (6) oder des Antriebselements (13).

5. Injektionsgerät nach Anspruch 3, **dadurch gekennzeichnet dass** der Trennvorgang eine axiale Lösebewegung umfasst und das Modulgehäuse (17) durch Schnappelemente (12b) verschnappt ist, welche von dem Freigabeelement (19) in der Sperrstellung am Lösen gehindert werden.

6. Injektionsgerät nach Anspruch 3, **dadurch gekennzeichnet dass** der Trennvorgang eine rotierende Lösebewegung umfasst und das Freigabeelement (19) in der Sperrstellung einen radialen Vorsprung (17a) am Modulgehäuse (17) am Rotieren hindert oder von einem radialen Vorsprung am Gerätegehäuse am Rotieren gehindert wird.

7. Injektionsgerät nach Anspruch 1, **dadurch gekennzeichnet, dass** das Elektronikmodul (16) aufweist eine Batterie (16b) zur Speisung einer Kommunikationseinheit des Elektronikmoduls (16), welche nur bei getrenntem Modulgehäuse (17) vom Elektronikmodul (16) entfernt werden kann.

8. Injektionsgerät nach Anspruch 1, umfassend eine Ausschüttfeder (9) zum Antrieb eines Vortriebselements (7) sowie ein Halteelement (6) zum Halten der Ausschüttfeder (9) in einem vorgespannten Zustand vor der Auslösung oder ein Antriebselement (13) zur Übertragung einer Ausschüttkraft der Ausschüttfeder (9) auf das Vortriebselement (7), **dadurch gekennzeichnet, dass** der Sensor (16a) eine durch die Ausschüttfeder (9) bewirkte axiale oder radiale Bewegung des Vortriebselements (7), des Halteelements (6), oder des Antriebselements (13) detektiert.

9. Injektionsgerät nach Anspruch 1, umfassend eine Nadelschutzfeder (10) zum Antrieb einer Nadelschutzhülse (3) nach distal und/oder eines Signalglieds (11) nach proximal, **dadurch gekennzeichnet, dass** der Sensor (16a) eine durch die Nadelschutzfeder (10) bewirkte axiale Bewegung der Nadelschutzhülse (3) oder des Signalglieds (11) detektiert.

10. Injektionsgerät nach Anspruch 1, **dadurch gekennzeichnet dass** das Injektionsgerät ein Autoinjektor ist mit einer maximal vorgespannten Ausschüttfeder (9) für eine einmalige Ausschüttung des gesamten oder zumindest eines vorbestimmten Inhalts einer Fertigspritze.

11. Ein erster Autoinjektor nach Anspruch 10 und ein zweiter Autoinjektor, der zweite Autoinjektor aufweisend ein zum ersten Autoinjektor identisches zweites Gerätegehäuse mit einer Öffnung an einem proximalen Ende, einen identischen Antrieb, und eine mit dem zweiten Gerätegehäuse unlösbar verbundene Abschlusskappe zur Abdeckung der Öffnung.

12. Der erste Autoinjektor nach Anspruch 11, **gekennzeichnet durch** einen Gehäuseeinsatz (12), welcher dreh- und axialfest mit dem proximalen Ende des Gerätegehäuses (2) des ersten Autoinjektors verbunden ist und als Moduladapter für das Modulgehäuse (17) mit dem Elektronikmodul (16) dient.
